# EUROPEAN PATENT APPLICATION

(11) **EP 4 539 088 A1**
(43) Date of publication of application: **16.04.2025**
(21) Application number: 22946948.1
(22) Date of filing: 14.06.2022
(51) Int. Cl.: H01J 35/18, H01J 35/04, H01J 35/06, A61B 6/06

(54) **X-RAY GENERATOR AND X-RAY SYSTEM USING SAME**

(71) Applicant: LG Electronics Inc., Yeongdeungpo-gu Seoul 07336 (KR)
(72) Inventor: KIM, Jinah, Seoul 06772 (KR); YIM, Jeongsoon, Seoul 06772 (KR); LEE, Byungkee, Seoul 06772 (KR); LEE, Giwon, Seoul 06772 (KR); NAM, Hyojin, Seoul 06772 (KR); HONG, Eunju, Seoul 06772 (KR); PARK, Hyungjo, Seoul 06772 (KR)
(74) Representative: Frenkel, Matthias Alexander
(86) International application number: PCT/KR2022/008424
(87) International publication number: WO 2023/243742

(57) **Abstract**

The present disclosure relates to an X-ray generating apparatus capable of providing an X-ray image and an X-ray system using the same, including: a field emitting part having a plurality of electron beam emitting regions arranged; an X-ray generating part generating X-rays by collision with electrons emitted from the field emitting part; and, a collimator transmitting X-rays generated from the X-ray generating part in a specific direction, in which a plurality of through-holes are arranged on the collimator to transmit the X-rays in a specific direction, and the X-ray can be blocked in a region other than the through-hole.

## Description

### [Technical Field]

The present disclosure relates to an X-ray generating apparatus capable of providing an X-ray image and an X-ray system using the same.

### [Background Art]

In general, an X-ray generating apparatus is widely used for medical diagnosis and non-destructive testing to reveal defects inside various structures.

The X-ray generating apparatus has a volume emitting source whose X-ray generating area has a specific volume.

In order for an X-ray generating apparatus with a volume emitting source to obtain a clear X-ray image, the size of the volume emitting source needs to be reduced as much as possible to be close to a point emitting source.

Therefore, an X-ray generating apparatus having a volume emitting source has a limitation in that it must not only use an electron focusing lens to reduce the size of the volume emitting source, but also secure a certain distance between the subject, the X-ray detector, and the emitting source in order to obtain a clear X-ray image.

In addition, the X-ray generating apparatus with volume emitting sources require a solution to the heat dissipation problem because as the size of the emitting source decreases, heat generation in the emitting source region increases rapidly.

In this way, an X-ray generating apparatus having a volume emitting source must have a small emitting source size, a large distance between the emitting source and the subject, and a small distance between the subject and the X-ray detector in order to obtain a clear X-ray image.

Due to these problems, in order for an X-ray system to operate, more than a certain amount of space is required, and the size of the emitting source must be made very small, so an electronic focusing lens must be added to the X-ray generating apparatus, and there is also the difficulty of having to solve the problem of heat generation in the emitting source region.

### [Disclosure]

### [Technical Problem]

An object of the present disclosure is to solve the problems described above and other problems.

An object of the present disclosure is to provide an X-ray generating apparatus which can provide a clear X-ray image without installation space constraints by emitting X-rays in a direction perpendicular to a surface using a collimator, and an X-ray system using the same.

In addition, an object of the present disclosure is to provide an X-ray generating apparatus capable of minimizing heat generation by dispersing energy required for obtaining an X-ray image using a collimator that transmits X-rays in a specific direction, and an X-ray system using the same.

In addition, an object of the present disclosure is to provide an X-ray generating apparatus which can reduce power consumption and minimize the probability of unnecessary X-ray exposure by individually driving field emission devices to inject X-rays only to a localized region corresponding to the shape of a subject, and an X-ray system using the same.

### [Technical Solution]

An X-ray generating apparatus according to one embodiment of the present disclosure includes a field emitting part having a plurality of electron beam emitting regions arranged; an X-ray generating part generating X-rays by collision with electrons emitted from the field emitting part, and, a collimator transmitting X-rays generated from the X-ray generating part in a specific direction, in which a plurality of through-holes may be arranged on the collimator to transmit the X-rays in a specific direction, and the X-ray may be blocked in a region other than the through-hole.

An X-ray generation system according to one embodiment of the present disclosure includes an X-ray generating apparatus generating and emitting X-rays; and, an X-ray detection device detecting X-rays emitted from the X-ray generating apparatus, in which the X-ray generating apparatus may includes a field emitting part having a plurality of electron beam emitting regions arranged; an X-ray generating part generating X-rays by collision with electrons emitted from the field emitting part; and, a collimator transmitting X-rays generated from the X-ray generating part in a specific direction, and a plurality of through-holes may be arranged on the collimator to transmit the X-rays in a specific direction, and the X-ray may be blocked in a region other than the through-hole.

### [Advantageous Effect]

According to one embodiment of the present disclosure, an X-ray generating apparatus and an X-ray system using the same can provide clear X-ray images without installation space constraints.

In addition, according to one embodiment of the present disclosure, the X-ray generating apparatus and the X-ray system using the same can disperse energy required to obtain an X-ray image, thereby minimizing heat generation.

In addition, according to one embodiment of the present disclosure, the X-ray generating apparatus and the X-ray system using the same can reduce power consumption and minimize the probability of unnecessary X-ray exposure by individually driving the field emission devices to inject X-rays only to a local region corresponding to the shape of the subject.

### [Description of Drawings]

FIGS. 1 to 3 are cross-sectional views illustrating an X-ray generating apparatus according to an embodiment of the present disclosure.
FIG. 4 is a perspective view illustrating a collimator of an X-ray generating apparatus according to one embodiment of the present disclosure.
FIGS. 5 to 7 are cross-sectional views illustrating a through-hole of a collimator according to an embodiment of the present disclosure.
FIGS. 8 to 10 are cross-sectional views illustrating the collimator thickness of an X-ray generating apparatus according to an embodiment of the present disclosure.
FIGS. 11 and 12 are cross-sectional views illustrating conditions for determining the through-hole size of a collimator according to one embodiment of the present disclosure.
FIG. 13 is a cross-sectional view illustrating a multi-layer collimator according to one embodiment of the present disclosure.
FIG. 14 is a view illustrating an X-ray system using an X-ray generating apparatus according to one embodiment of the present disclosure.

### [Best Mode]

Hereinafter, embodiments disclosed in this specification will be described in detail with reference to the attached drawings, wherein, regardless of the drawing symbols, identical or similar components will be given the same reference numerals and redundant descriptions thereof will be omitted. The suffixes "module" and "part" used for components in the following description are assigned or used interchangeably only for the convenience of writing the specification, and do not have distinct meanings or roles in themselves. In addition, when describing embodiments disclosed in this specification, if it is determined that a specific description of a related known technology may obscure the gist of the embodiments disclosed in this specification, the detailed description thereof will be omitted. In addition, the attached drawings are only intended to facilitate easy understanding of the embodiments disclosed in this specification, and the technical ideas disclosed in this specification are not limited by the attached drawings, and should be understood to include all modifications, equivalents, and substitutes included in the idea and technical scope of the present disclosure.

Terms including ordinal numbers, such as first, second, and the like may be used to describe various components, but the components are not limited by the terms. The terms are used only to distinguish one component from another.

When it is said that a component is "connected" or "accessed" to another component, it should be understood that it may be directly connected or accessed to that other component, but that there may be other components in between. On the other hand, when it is said that a component is "directly connected" or "directly accessed" to another component, it should be understood that there are no other components in between.

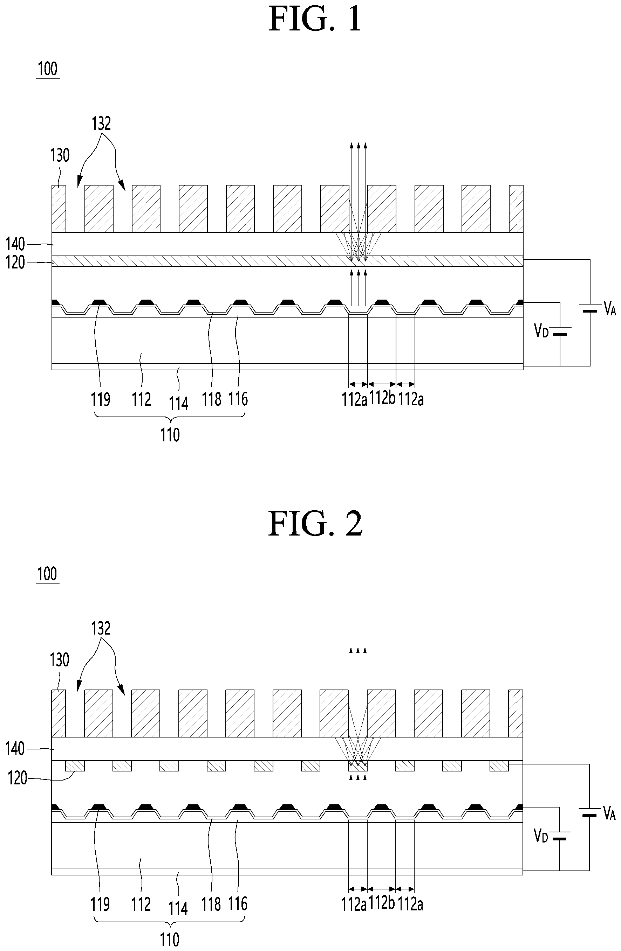

FIG. 1 is a cross-sectional view illustrating an X-ray generating apparatus according to an embodiment of the present disclosure.

As illustrated in FIG. 1, the X-ray generating apparatus 100 of the present disclosure may include a field emitting part 110 in which a plurality of electron beam emitting regions 112a are arranged, an X-ray generating part 120 that generates X-rays by collision with electrons emitted from the field emitting part 110, and a collimator 130 that transmits the X-rays generated from the X-ray generating part 120 in a specific direction.

Here, the collimator 130 has a plurality of through-holes 132 arranged to transmit X-rays in a specific direction, and can block X-rays in areas other than the through-holes 132.

For example, the collimator 130 may be composed of a material that does not scatter or reflect and may have a grid shape.

In addition, the collimator 130 can also be used as a heat dissipation structure that disperses and releases high temperature heat generated from the X-ray generating part 120.

Next, the through-hole 132 of the collimator 130 can be arranged corresponding to the electron beam emitting region 112a of the field emitting part 110.

Here, the central axis of the through-hole 132 of the collimator 130 may be positioned on the same line as the central axis of the electron beam emitting region 112a of the field emitting part 110.

Additionally, the number of through-holes 132 of the collimator 130 may be the same as the number of electron beam emitting regions 112a of the field emitting part 110.

Here, the through-holes 132 of the collimator 130 can be arranged so that one through-hole 132 corresponds one to one in one electron beam emitting region 112a.

In other words, when one electron beam emitting region 112a has a dot shape, the through-hole 132 of the collimator 130 can be arranged so that one through-hole 132 corresponds to each dot-shaped electron beam emitting region 112a.

In some cases, the number of through-holes 132 of the collimator 130 may be greater than the number of electron beam emitting regions 112a of the field emitting part 110.

Here, the through-holes 132 of the collimator 130 can be arranged so that a plurality of through-holes 132 correspond to one electron beam emitting region 112a.

In other words, when one electron beam emitting region 112a has a stripe shape, a plurality of through-holes 132 of the collimator 130 can be arranged in a row along the electron beam emitting region 112a of the stripe shape.

Additionally, the through-hole 132 of the collimator 130 may have an angle of the inner surface that is perpendicular to the upper or lower surface of the collimator 130.

In some cases, the through-hole 132 of the collimator 130 may have an angle of the inner surface that is perpendicular to the surface of the X-ray generating part 120.

Additionally, the collimator 130 can determine the transmission direction of the X-ray according to the inner surface angle of the through-hole 132.

Here, the collimator 130 can determine the inner surface angle of the through-hole so that the transmission direction of the X-ray is perpendicular to the upper surface or lower surface of the collimator 130.

In some cases, the collimator 130 may determine the inner surface angle of the through-hole 132 so that the transmission direction of the X-ray is perpendicular to the surface of the X-ray generating part 110.

Additionally, the X-ray passing through the collimator 130 can have a transmission direction parallel to the inner surface of the through-hole 132.

In addition, the through-hole 132 of the collimator 130 may include an upper region facing the subject and a lower region facing the X-ray generating part 120, and the area of the upper region and the area of the lower region may be equal to each other.

Here, when the through-holes 132 of the collimator 130 are arranged in a one-to-one correspondence with the electron beam emitting region 112a of the field emitting part 110, the area of the lower region of each through-hole 132 may be equal to or larger than the area of the electron beam emitting region 112a corresponding to each through-hole 132.

Additionally, the collimator 130 may be disposed in contact with the X-ray generating part 120.

Here, the thickness of the collimator 130 may be thicker than the thickness of the X-ray generating part 120.

At this time, the collimator 130 may include a heat-conducting material that emits heat generated from the X-ray generating part 120 and an absorbing material that absorbs X-rays generated from the X-ray generating part 120.

In some cases, the collimator 130 may be disposed at a certain distance from the X-ray generating part 110.

Here, the thickness of the collimator 130 may be thicker than the gap between the X-ray generating part 120 and the collimator 130.

For example, the gap between the X-ray generating part 120 and the collimator 130 may be smaller than the thickness of the collimator 130 and larger than the thickness of the X-ray generating part 120.

At this time, the collimator 130 may include an absorbing material that absorbs X-rays generated from the X-ray generating part 120.

In another case, a transmission window 140 that transmits X-rays may be disposed between the collimator 130 and the X-ray generating part 120.

Here, the lower surface of the transmission window 140 can be in contact with the X-ray generating part 120, and the upper surface of the transmission window 140 can be in contact with the collimator 130.

At this time, the thickness of the collimator 130 may be thicker than the thickness of the transmission window 140.

For example, the thickness of the transmission window 140 may be thinner than the thickness of the collimator 130 and thicker than the thickness of the X-ray generating part 120.

The collimator 130 placed above the transmission window 140 may include a heat-conducting material that emits heat generated from the X-ray generating part 120 and an absorbing material that absorbs X-rays generated from the X-ray generating part 120.

Additionally, the through-hole 132 of the collimator 130 may occupy an area of 40% to 90% of the total area of the collimator 130.

This is because, if the area of the through-hole 132 is too small, the amount of X-ray emission may be small and the X-ray image may become too dark, and if the area of the through-hole 132 is too large, the amount of X-ray emission may be large and the X-ray image may become too bright.

Additionally, the area ratio of the through-hole 132 occupying the collimator 130 can be determined corresponding to the area ratio of the electron beam emitting region 112a occupying the field emitting part 110.

For example, the area ratio of the through-hole 132 occupying the collimator 130 can increase or decrease in proportion to the area ratio of the electron beam emitting region 112a occupying the field emitting part 110.

In addition, the size of the through-hole 132 of the collimator 130 can be determined corresponding to at least one of the first condition for the size of the electron beam emitting region 112a of the field emitting part 110 and the second condition for the thickness of the collimator 130.

Here, the size of the through-hole 132 of the collimator 130 can increase or decrease in proportion to the size of the electron beam emitting region 112a of the field emitting part 110.

In some cases, the size of the through-hole 132 of the collimator 130 may increase or decrease in proportion to the thickness of the collimator 130.

Additionally, the through-holes 132 of the collimator 130 are arranged in a matrix form, and the spacing between the through-holes 132 can be the same.

Here, the through-holes 132 of the collimator 130 have the same plane shape and may have at least one shape among a circle, a square, and a polygon, but this is only an example and is not limited thereto.

Additionally, the thickness of the collimator 130 can be determined corresponding to the resolution of the X-ray image to be obtained.

Here, the thickness of the collimator 130 can be increased or decreased in proportion to the resolution of the X-ray image.

Additionally, the collimator 130 may include a first collimator disposed adjacent to the X-ray generating part 120 and having a plurality of first through-holes arranged therein, and at least one second collimator disposed above the first collimator and having a plurality of second through-holes arranged therein.

Here, the second through-holes can be arranged to correspond to the first through-holes, respectively.

At this time, the second collimator can be laminated by coming into contact with the first collimator.

The number of laminated layer of the second collimator can be determined in accordance with the resolution of the X-ray image to be obtained.

For example, the number of laminated layer of second collimators can be increased or decreased in proportion to the resolution of the X-ray image.

In addition, the thickness of the second collimator can be the same as the thickness of the first collimator.

In some cases, the thickness of the second collimator may be thicker than the thickness of the first collimator.

Here, the thickness of the second collimator can be determined corresponding to the resolution of the X-ray image to be obtained.

In other words, the thickness of the second collimator can be increased or decreased in proportion to the resolution of the X-ray image.

Additionally, the size of the second through-hole may be the same as the size of the first through-hole.

Here, the central axis of the second through-hole may be positioned on the same line as the central axis of the first through-hole, and the central axes of the first and second through-holes may be positioned on the same line as the central axis of the electron beam emitting region 112a of the field emitting part 110.

Next, the X-ray generating part 120 may include a transmission type anode.

Here, the X-ray generating part 120 can be disposed at a certain interval so as to have a certain space from the field emitting part 110.

Additionally, a predetermined space between the X-ray generating part 120 and the field emitting part 110 may be in a vacuum state.

Next, the X-ray generating part 120 can be disposed to cover the entire area of the field emitting part 110.

In some cases, the X-ray generating part 120 may be positioned to cover only a part of the area of the field emitting part 110.

Here, the X-ray generating part 120 can be arranged to cover only the electron beam emitting regions 112a of the field emitting part 110.

Additionally, the X-ray generating part 120 may be disposed so that a plurality of transmission type anodes are separated and spaced apart from each other.

Here, the transmission type anode can be arranged corresponding to the electron beam emitting region 112a of the field emitting part 110.

Next, the field emitting part 110 may include a semiconductor substrate 112 including a plurality of electron beam emitting regions 112a and electron beam non-emitting regions 112b, a bottom electrode 114 disposed under the semiconductor substrate 112, an insulating layer 116 disposed over the semiconductor substrate 112, a gate electrode 118 disposed on the insulating layer 116, and a top electrode 119 disposed on the gate electrode 118 located in the electron beam non-emitting region 112b.

Here, the bottom electrode 114 can be electrically connected in parallel to the top electrode 119 and the transmission type anode of the X-ray generating part 120.

In addition, the thickness of the insulating layer 116 positioned in the electron beam emitting region 112a of the semiconductor substrate 112 may be thinner than the thickness of the insulating layer 116 positioned in the electron beam non-emitting region 112b of the semiconductor substrate 112.

In this way, when the X-ray generating apparatus 100 of the present disclosure has a field emitting part 110 disposed at the lower part having a MIS (Metal-Insulator-Semiconductor) or MIM (Metal-Insulator-Metal) structure that emits electrons, and an X-ray generating part 120 including a transmission type anode and a transmission window 140 disposed at the upper part, the space between them can be in a vacuum state of about 1×10⁻³ to about 1×10⁻³ Torr.

In addition, in the X-ray generating apparatus 100 of the present disclosure, if a diode voltage V_{D} is applied to the bottom electrode 114 and the top electrode 119 of the field emitting part 110, electrons can be emitted from the electron beam emitting region 112a of the semiconductor substrate 112 into a vacuum space.

In addition, the emitted electrons can be accelerated by the anode voltage V_{A} applied to the transmission type anode of the X-ray generating part 120 and collide with the transmission type anode to generate X-rays.

Here, the transmission type anode of the X-ray generating part 120 may be composed of an X-ray generating material such as tungsten or diamond in a thin film form, as the generated X-rays must be transmitted, and the transparent window 140 positioned above the X-ray generating part 120 may be composed of a material such as beryllium through which X-rays can be transmitted well.

Additionally, X-rays generated by colliding with a transmission type anode can be emitted in an omni-direction.

In this way, since X-rays generated by colliding with a transmission type anode are emitted in omni-direction, the present disclosure can only pass X-rays directed in a direction perpendicular to a flat surface by using a collimator 130.

In other words, the collimator 130 of the present disclosure has a plurality of through-holes 132 arranged to transmit X-rays in a direction perpendicular to the surface, and can block X-rays in a region other than the through-holes 132.

The collimator 130 may be composed of a material that does not scatter or reflect and may have a grid shape.

In addition, the collimator 130 can also be used as a heat dissipation structure that disperses and releases high temperature heat generated from the X-ray generating part 120.

In this way, the present disclosure can provide a clear X-ray image without installation space constraints by emitting X-rays in a direction perpendicular to the surface using a collimator.

In addition, the present disclosure can minimize heat generation by dispersing energy required to obtain an X-ray image using a collimator that transmits X-rays in a specific direction.

In addition, the present disclosure can reduce power consumption and minimize the probability of unnecessary X-ray exposure by individually driving field emission devices to inject X-rays only to a local region corresponding to the shape of the subject.

FIG. 2 is a cross-sectional view illustrating an X-ray generating apparatus according to another embodiment of the present disclosure.

As illustrated in FIG. 2, the X-ray generating apparatus 100 of the present disclosure may include a field emitting part 110 in which a plurality of electron beam emitting regions 112a are arranged, an X-ray generating part 120 that generates X-rays by collision with electrons emitted from the field emitting part 110, and a collimator 130 that transmits the X-rays generated from the X-ray generating part 120 in a specific direction.

Here, the X-ray generating part 120 may be positioned to cover only a part of the area of the field emitting part 110.

In other words, the X-ray generating part 120 can be disposed to cover only the electron beam emitting regions 112a of the field emitting part 110.

The X-ray generating part 120 may be arranged such that a plurality of transmission type anodes are separated and arranged to have a regular interval.

Here, the transmission type anode can be arranged corresponding to the electron beam emitting region 112a of the field emitting part 110 and the through-hole 132 of the collimator 130.

Additionally, the X-ray generating part 120 may have a plurality of transmission type anodes arranged at regular intervals on the lower surface of the transmission window 140.

In addition, the collimator 130 can have a plurality of through-holes 132 arranged corresponding to the transmission type anode on the upper surface of the transparent window 140.

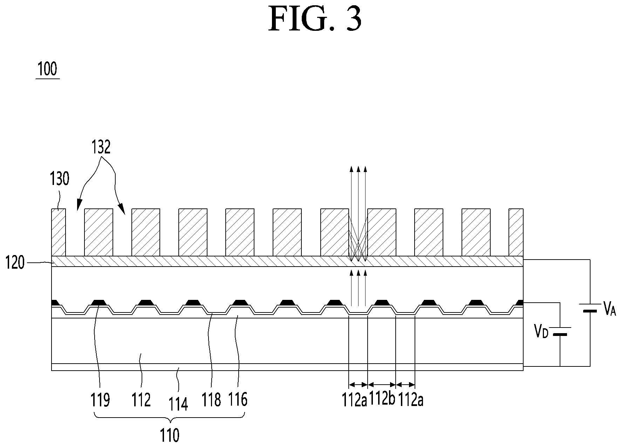

FIG. 3 is a cross-sectional view illustrating an X-ray generating apparatus according to another embodiment of the present disclosure.

As illustrated in FIG. 3, the X-ray generating apparatus 100 of the present disclosure may include a field emitting part 110 in which a plurality of electron beam emitting regions 112a are arranged, an X-ray generating part 120 that generates X-rays by collision with electrons emitted from the field emitting part 110, and a collimator 130 that transmits the X-rays generated from the X-ray generating part 120 in a specific direction.

Here, the collimator 130 can be disposed on the X-ray generating part 120 while being in contact with the X-ray generating part.

The reason is that it is advantageous for a miniaturized structure because the overall thickness of the X-ray generating apparatus 100 can be reduced.

Additionally, the thickness of the collimator 130 may be thicker than the thickness of the X-ray generating part 120.

The reason is to allow only X-rays emitted in a direction perpendicular to the panel surface to pass through.

Additionally, the collimator 130 may include a heat-conducting material that emits heat generated from the X-ray generating part 120 and an absorbing material that absorbs X-rays generated from the X-ray generating part 120.

The reason is that only X-rays emitted in a direction perpendicular to the panel surface are transmitted, and the high temperature generated in the X-ray generating part 120 is used to disperse and release heat.

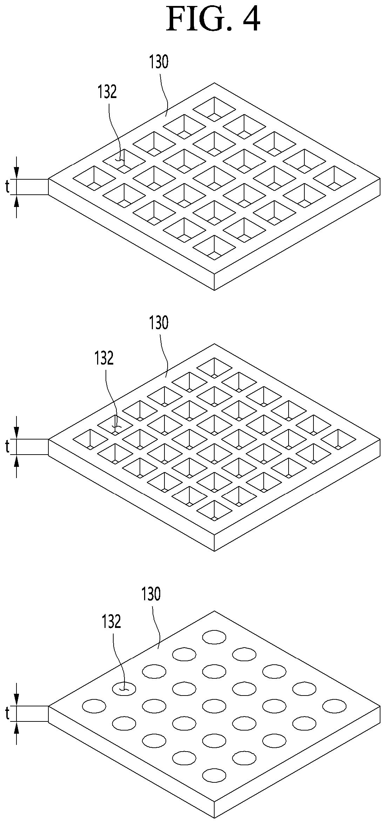

FIG. 4 is a perspective view illustrating a collimator of an X-ray generating apparatus according to one embodiment of the present disclosure.

As illustrated in Fig. 4, the collimator 130 may have a plurality of through-holes 132 arranged to transmit X-rays in a vertical direction.

Additionally, the collimator 130 can block X-rays in an area other than the through-hole 132.

For example, the collimator 130 may be composed of a material that does not scatter or reflect and may have a grid shape.

Additionally, the collimator 130 can also be used as a heat dissipation structure that disperses and releases high temperature heat generated from the X-ray generating part 120.

The number of through-holes 132 of the collimator 130 may be the same as the number of electron beam emitting regions of the field emitting part.

Here, the through-holes 132 of the collimator 130 can be arranged so that one through-hole corresponds one-to-one to one electron beam emitting region.

In some cases, the number of through-holes 132 of the collimator 130 may be greater than the number of electron beam emitting regions of the field emitting part.

Here, the through-holes 132 of the collimator 130 can be arranged so that a plurality of through-holes correspond to one electron beam emitting region.

Additionally, the through-holes 132 of the collimator 130 are arranged in a matrix form, and the spacing between the through-holes 132 can be the same.

Here, the through-holes 132 of the collimator 130 have the same plane shape and may have at least one shape among a circle, a square, and a polygon, but this is only an example and is not limited thereto.

Additionally, the thickness t of the collimator 130 can be determined corresponding to the resolution of the X-ray image to be obtained.

Here, the thickness t of the collimator 130 can increase or decrease in proportion to the resolution of the X-ray image.

Additionally, the through-hole 132 of the collimator 130 may occupy an area of 40% to 90% of the total area of the collimator 130.

This is because, if the area of the through-hole 132 is too small, the amount of X-ray emission may be small and thus the X-ray image may become too dark, and if the area of the through-hole 132 is too large, the amount of X-ray emission may be large and thus the X-ray image may become too bright.

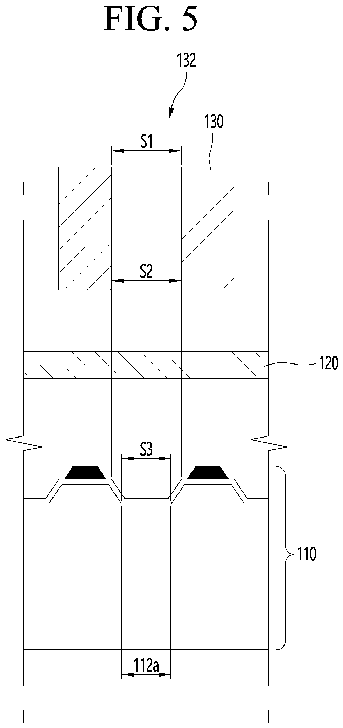

FIGS. 5 to 7 are cross-sectional views illustrating a through-hole of a collimator according to an embodiment of the present disclosure.

As illustrated in FIG. 5, the through-hole 132 of the collimator 130 may include an upper region facing the subject and a lower region facing the X-ray generating part 120, and the area S1 of the upper region and the area S2 of the lower region may be equal to each other.

Here, when the through-holes 132 of the collimator 130 are arranged in a one-to-one correspondence with the electron beam emitting regions 112a of the field emitting part 110, the area S2 of the lower region of each through-hole 132 may be equal to or larger than the area S3 of the electron beam emitting region 112a corresponding to each through-hole 132.

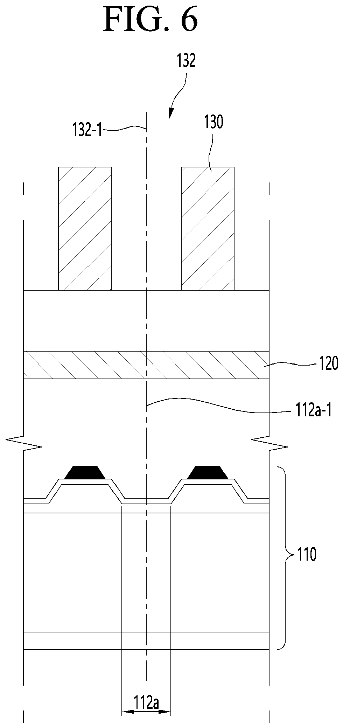

Additionally, as illustrated in FIG. 6, the through-hole 132 of the collimator 130 can be arranged corresponding to the electron beam emitting region 112a of the field emitting part 110.

Here, the central axis 132-1 of the through-hole 132 of the collimator 130 may be positioned on the same line as the central axis 112a-1 of the electron beam emitting region 112a of the field emitting part 110.

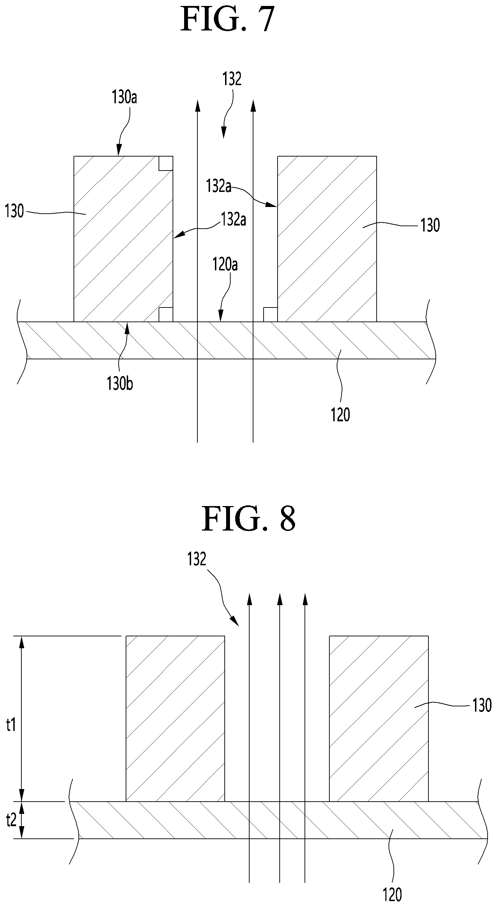

In addition, as illustrated in FIG. 7, the through-hole 132 of the collimator 130 may have an angle of the inner surface 132a that is perpendicular to the upper surface 130a or the lower surface 130b of the collimator 130.

In some cases, the through-hole 132 of the collimator 130 may have an angle of the inner surface 132a that is perpendicular to the surface 120a of the X-ray generating part 120.

Additionally, the collimator 130 can determine the transmission direction of the X-ray according to the angle of the inner surface 132a of the through-hole 132.

Here, the collimator 130 can determine the angle of the inner surface 132a of the through-hole 132 so that the transmission direction of the X-ray is perpendicular to the upper surface 130a or the lower surface 130b of the collimator 130.

In some cases, the collimator 130 may determine the angle of the inner surface 132a of the through-hole 132 so that the transmission direction of the X-ray is perpendicular to the surface 120a of the X-ray generating part 110.

Additionally, the X-ray passing through the collimator 130 can have a transmission direction parallel to the inner surface 132a of the through-hole 132.

FIGS. 8 to 10 are cross-sectional views illustrating the collimator thickness of an X-ray generating apparatus according to an embodiment of the present disclosure.

As illustrated in Fig. 8, the collimator 130 can be placed in contact with the X-ray generating part 120.

Here, the thickness t1 of the collimator 130 may be thicker than the thickness t2 of the X-ray generating part 120.

At this time, the collimator 130 may include a heat-conducting material that emits heat generated from the X-ray generating part 120 and an absorbing material that absorbs X-rays generated from the X-ray generating part 120.

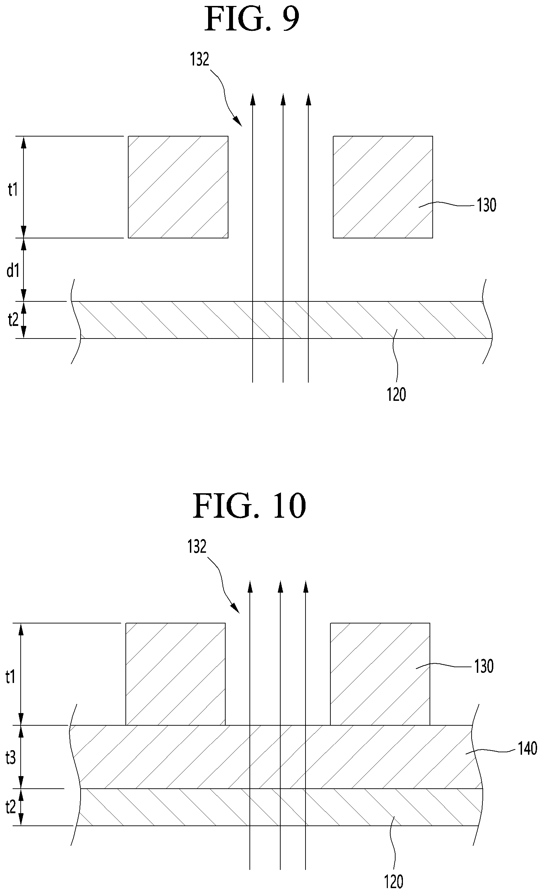

In some cases, as illustrated in FIG. 9, the collimator 130 may be disposed at a certain distance from the X-ray generating part 110.

Here, the thickness t1 of the collimator 130 may be thicker than the gap d1 between the X-ray generating part 120 and the collimator 130.

For example, the distance d1 between the X-ray generating part 120 and the collimator 130 may be smaller than the thickness t1 of the collimator 130 and larger than the thickness t2 of the X-ray generating part 120.

At this time, the collimator 130 may include an absorbing material that absorbs X-rays generated from the X-ray generating part 120.

In another case, as illustrated in FIG. 10, a transmission window 140 that transmits X-rays may be placed between the collimator 130 and the X-ray generating part 120.

Here, the lower surface of the transmission window 140 can be in contact with the X-ray generating part 120, and the upper surface of the transmission window 140 can be in contact with the collimator 130.

At this time, the thickness t1 of the collimator 130 may be thicker than the thickness t3 of the transmission window 140.

For example, the thickness t3 of the transmission window 140 may be thinner than the thickness t1 of the collimator 130 and thicker than the thickness t2 of the X-ray generating part 120.

The collimator 130 placed above the transmission window 140 may include a heat-conducting material that emits heat generated from the X-ray generating part 120 and an absorbing material that absorbs X-rays generated from the X-ray generating part 120.

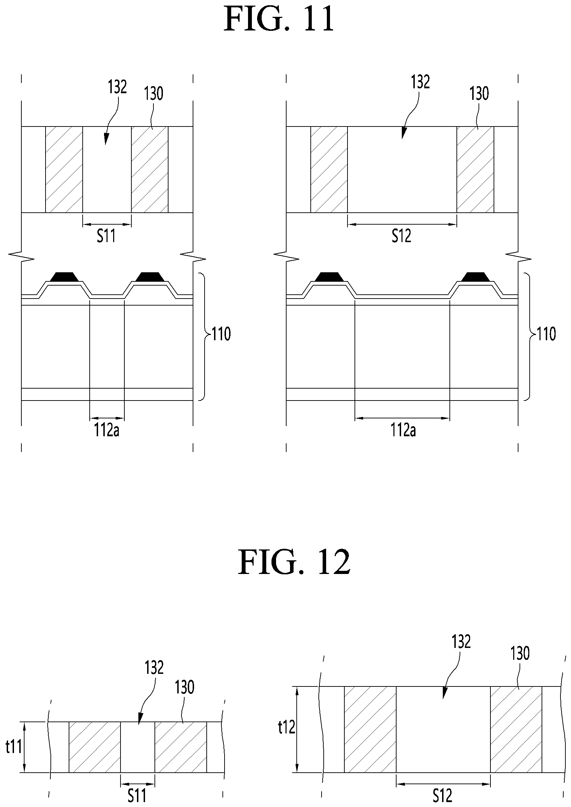

FIGS. 11 and 12 are cross-sectional views illustrating conditions for determining the through-hole size of a collimator according to one embodiment of the present disclosure.

As illustrated in FIG. 11, the area ratio of the through-hole 132 occupying the collimator 130 can be determined corresponding to the area ratio of the electron beam emitting region 112a occupying the field emitting part 110.

For example, the area ratio of the through-hole 132 occupying the collimator 130 can increase or decrease in proportion to the area ratio of the electron beam emitting region 112a occupying the field emitting part 110.

In other words, if the area of the electron beam emitting region 112a of the field emitting part 110 is small, the area S11 of the through-hole 132 of the collimator 130 can be formed correspondingly small, and if the area of the electron beam emitting region 112a of the field emitting part 110 is large, the area S12 of the through-hole 132 of the collimator 130 can be formed correspondingly large.

The reason is to secure as much as possible without loss the X-rays emitted in a direction perpendicular to the panel surface.

In this way, the size of the through-hole 132 of the collimator 130 can be determined in accordance with the condition for the size of the electron beam emitting region 112a of the field emitting part 110.

Here, the size of the through-hole 132 of the collimator 130 can increase or decrease in proportion to the size of the electron beam emitting region 112a of the field emitting part 110.

Additionally, as illustrated in FIG. 12, the area ratio of the through-hole 132 occupying the collimator 130 can be determined corresponding to the thickness ratio of the collimator 130.

For example, the area ratio of the through-hole 132 occupying the collimator 130 can increase or decrease in proportion to the thickness ratio of the collimator 130.

In other words, if the thickness t11 of the collimator 130 is thin, the area S11 of the through-hole 132 of the collimator 130 can be formed correspondingly smaller, and if the thickness t12 of the collimator 130 is thick, the area S12 of the through-hole 132 of the collimator 130 can be formed correspondingly larger.

The reason is to secure as much as possible without loss the X-rays emitted in a direction perpendicular to the panel surface.

In this way, the size of the through-hole 132 of the collimator 130 can be determined corresponding to the condition for the thickness of the collimator 130.

Here, the size of the through-hole 132 of the collimator 130 can increase or decrease in proportion to the thickness of the collimator 130.

In some cases, the size of the through-hole 132 of the collimator 130 may be determined in accordance with the first condition for the size of the electron beam emitting region 112a of the field emitting part 110 and the second condition for the thickness of the collimator 130.

Here, the size of the through-hole 132 of the collimator 130 can increase or decrease in proportion to the size of the electron beam emitting region 112a of the field emitting part 110 and the thickness of the collimator 130.

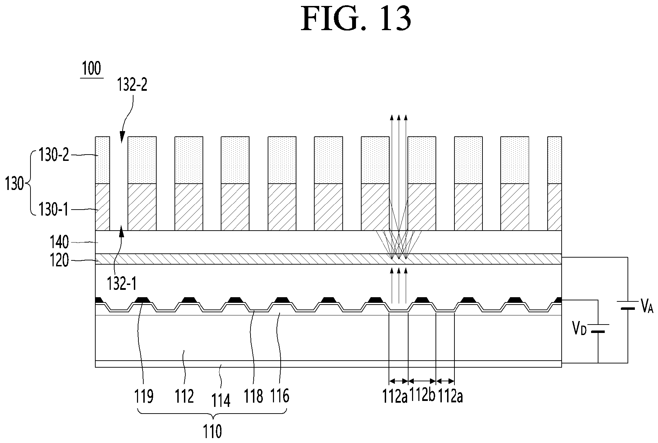

FIG. 13 is a cross-sectional view illustrating a multi-layer collimator according to one embodiment of the present disclosure.

As illustrated in FIG. 13, the collimator 130 may include a first collimator 130-1 disposed adjacent to the X-ray generating part 120 and having a plurality of first through-holes 132-1 arranged therein, and at least one second collimator 130-2 arranged above the first collimator 130-1 and having a plurality of second through-holes 132-2 arranged therein.

Here, the second through-holes 132-2 can be arranged to correspond to the first through-holes 132-1.

At this time, the second collimator 130-2 can be laminated by coming into contact with the first collimator 130-1.

The number of laminated layer of the second collimator 130-2 can be determined in accordance with the resolution of the X-ray image to be obtained.

For example, the number of laminated layer of second collimators 130-2 can be increased or decreased in proportion to the resolution of the X-ray image.

In addition, the thickness of the second collimator 130-2 may be the same as the thickness of the first collimator 130-1.

In some cases, the thickness of the second collimator 130-2 may be thicker than the thickness of the first collimator 130-1.

Here, the thickness of the second collimator 130-2 can be determined corresponding to the resolution of the X-ray image to be obtained.

In other words, the thickness of the second collimator 130-2 can be increased or decreased in proportion to the resolution of the X-ray image.

Additionally, the size of the second through-hole 132-2 may be the same as the size of the first through-hole 132-1.

Here, the central axis of the second through-hole 132-2 may be positioned on the same line with respect to the central axis of the first through-hole 132-1, and the central axes of the first and second through-holes 132-1, 132-2 may be positioned on the same line with respect to the central axis of the electron beam emitting region 112a of the field emitting part 110.

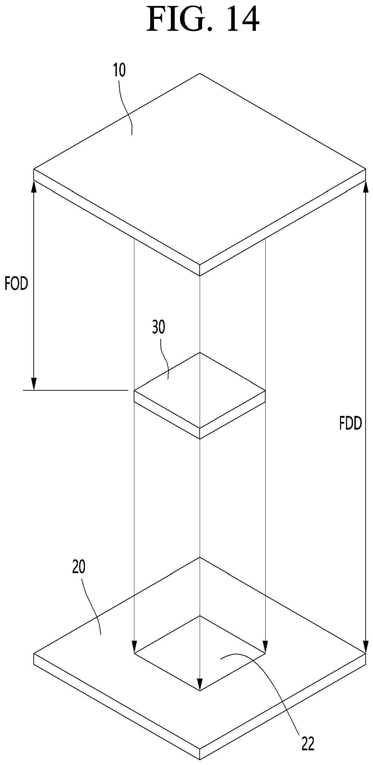

FIG. 14 is a view illustrating an X-ray system using an X-ray generating apparatus according to one embodiment of the present disclosure.

As illustrated in FIG. 14, the X-ray system may include an X-ray generating apparatus 10 that generates and emits X-rays, and an X-ray detecting device 20 that detects X-rays emitted from the X-ray generating apparatus 10.

Here, the X-ray generating apparatus 10 may include a field emitting part in which a plurality of electron beam emitting regions are arranged, an X-ray generating part that generates X-rays by collision with electrons emitted from the field emitting part, and a collimator that transmits X-rays generated from the X-ray generating part in a specific direction.

A collimator has a plurality of through-holes arranged to transmit X-rays in a specific direction, and can block the X-rays in regions other than the through-holes.

The X-ray generating apparatus 10 can emit X-rays in a direction perpendicular to the surface through a collimator.

Here, the through-holes of the collimator can be arranged corresponding to the electron beam emitting region of the field emitting part.

The X-ray system of the present disclosure utilizes an X-ray generating apparatus 10 that emits X-rays in the vertical direction of the panel, and has a structure that can disperse the tube current required for an X-ray image to a large area of the X-ray generating apparatus 10, so that a clear X-ray image 22 can be implemented and excellent reliability can be secured.

In addition, in conventional reflection type or transmission type X-ray generating apparatus, electron focusing lenses are used so that electrons are focused and collide on a local region of the anode, which causes deformation or damage to the anode when the tube current is increased, and a separate heat dissipation structure is required due to high heat generation.

However, the X-ray system of the present disclosure can obtain a clear and distortion-free X-ray image 22 because uniform X-rays are emitted in the vertical direction from the X-ray generating apparatus 10.

Accordingly, the X-ray system of the present disclosure can minimize the distance FDD between the X-ray generating apparatus 10 and the X-ray detecting device 20 as long as there is no size constraint of the subject 30, thereby minimizing the installation space of the X-ray system.

In addition, the X-ray system of the present disclosure is structured to disperse the energy required to obtain an X-ray image 22 to a wide area of an X-ray generating apparatus 10, and thus is free from heat generation problems compared to an X-ray generating apparatus of an electronic focusing structure, and therefore does not require a separate heat dissipation structure.

In addition, the X-ray system of the present disclosure can scan X-rays only to a local region when the field emission devices of the X-ray generating apparatus 10 are individually driven according to the shape of the subject 30, thereby reducing the power consumption of the X-ray generating apparatus 10 and also reducing the probability of exposure to unnecessary X-rays.

### [Industrial applicability]

According to the X-ray generating apparatus according to the present disclosure, a clear X-ray image can be provided without installation space constraints by using a collimator that transmits X-rays in a specific direction, and energy required to obtain an X-ray image can be dispersed, thereby minimizing heat generation, so the device has remarkable industrial applicability.

## Claims

1. An X-ray generating apparatus comprising:
a field emitting part having a plurality of electron beam emitting regions arranged,
an X-ray generating part generating X-rays by collision with electrons emitted from the field emitting part, and,
a collimator transmitting X-rays generated from the X-ray generating part in a specific direction,
wherein a plurality of through-holes are arranged on the collimator to transmit the X-rays in a specific direction, and the X-ray is blocked in a region other than the through-hole.

2. The X-ray generating apparatus of claim 1,
wherein the through-hole of the collimator is arranged corresponding to the electron beam emitting region of the field emitting part.

3. The X-ray generating apparatus of claim 1,
wherein an angle of an inner surface of the through-hole of the collimator is perpendicular to an upper surface or a lower surface of the collimator.

4. The X-ray generating apparatus of claim 1,
wheiren the collimator determines a transmission direction of the X-ray according to the angle of the inner surface of the through-hole.

5. The X-ray generating apparatus of claim 4,
wherein the collimator determines the angle of the inner surface of the through-hole so that the transmission direction of the X-ray is perpendicular to an upper surface or a lower surface of the collimator.

6. The X-ray generating apparatus of claim 1,
wherein the through-hole of the collimator includes:
an upper region facing a subject; and,
a lower region facing the X-ray generating part, and
wherein an area of the upper region and an area of the lower region are equal to each other.

7. The X-ray generating apparatus of claim 1,
wherein the collimator is placed on the X-ray generating part while being contact with the X-ray generating part.

8. The X-ray generating apparatus of claim 1, further includes:
a transmission window disposed between the collimator and the X-ray generating part to transmit the X-ray,
wherein a lower surface of the transmission window is in contact with the X-ray generating part, and
wherein an upper surface of the transmission window is in contact with the collimator.

9. The X-ray generating apparatus of claim 1,
wherein the through-holes of the collimator are arranged in matrix form, and
wherein the spacing between the through-holes is the same.

10. The X-ray generating apparatus of claim 1,
wherein the thickness of the collimator is determined corresponding to the resolution of an X-ray image to be obtained.

11. The X-ray generating apparatus of claim 1,
wherein the collimator includes:
a first collimator disposed adjacent to the X-ray generating part and having a plurality of first through-holes arranged therein; and
at least one second collimator disposed above the first collimator and having a plurality of second through-holes arranged therein; and
wherein the second through-hole is arranged to correspond to the first through-holes.

12. The X-ray generating apparatus of claim 1,
wherein the X-ray generating part includes a transmission type anode.

13. The X-ray generating apparatus of claim 1,
wherein the field emitting part includes:
a semiconductor substrate including a plurality of electron beam emitting regions and electron beam non-emitting regions;
a bottom electrode disposed below the semiconductor substrate;
an insulating layer disposed above the semiconductor substrate;
a gate electrode disposed on the insulating layer; and,
a top electrode disposed on a gate electrode located in the electron beam non-emitting region.

14. An X-ray system comprising:
an X-ray generating apparatus generating and emitting X-rays; and,
an X-ray detection device detecting X-rays emitted from the X-ray generating apparatus,
wherein the X-ray generating apparatus includes:
a field emitting part having a plurality of electron beam emitting regions arranged,
an X-ray generating part generating X-rays by collision with electrons emitted from the field emitting part; and,
a collimator transmitting X-rays generated from the X-ray generating part in a specific direction, and
wherein a plurality of through-holes are arranged on the collimator to transmit the X-rays in a specific direction, and the X-ray is blocked in a region other than the through-hole.

15. An X-ray system of claim 14,
wherein the X-ray generating apparatus emits X-rays in a direction perpendicular to a surface through the collimator.
